# EUROPEAN PATENT SPECIFICATION

(11) **EP 0 896 536 B1**
(45) Date of publication and mention of the grant of the patent: **12.01.2000**
(21) Application number: 97928360.3
(22) Date of filing: 24.06.1997
(51) Int. Cl.: A61K 31/415

(54) **USE OF LOFEXIDINE IN THE MANUFACTURE OF A MEDICAMENT FOR TREATING ATTENTION DEFICIT HYPERACTIVE DISORDER**
VERWENDUNG VON LOFEXIDIN ZUR HERSTELLUNG EINES ARZNEIMITTELS ZUR BEHANDKUNG DER MIT KONZENTRATIONSSCHWÄCHE EINHERGEHENDEN HYPERAKTIVITÄT
UTILISATION DE LOFEXIDINE DANS LA FABRICATION D'UN MEDICAMENT PERMETTANT DE TRAITER L'HYPERACTIVITE AVEC DEFICIT DE L'ATTENTION

(30) Priority: 25.06.1996 GB 9613243
(43) Date of publication of application: 17.02.1999
(73) Proprietor: BRITANNIA PHARMACEUTICALS LIMITED, Redhill, Surrey RH1 6YS (GB)
(72) Inventor: ARNSTEN, Amy, Bethany, CT 06524 (US); DAVIES, Keith, West Wickham, Kent BR4 9DJ (GB)
(74) Representative: Woodcraft, David Charles
(86) International application number: GB9701713
(87) International publication number: WO9749396

(56) References cited:
- EP-A- 0 721 777
- US-A- 4 800 209
- A.L. ROSTAIN: "Attention Deficit Disorder in Children and adolescents" PEDIATRIC CLINICS OF NORTH AMERICA, vol. 38, no. 3, 1991, pages 607-35, XP002043760

## Description

ADHD is a condition affecting a significant proportion of children and which is manifest by learning difficulties, restlessness, inability to settle to any task, argumentativeness, low frustration tolerance and aggressive conduct. In the past, a traditional method of treating such children was by administration of psycho stimulant such as methyl phenidate. While psycho stimulants are useful in increasing attention spans, they have major side-effects, including loss of appetite and insomnia and do not deal with the problems of hyperactivity.

More recently, clonidine has been tested as a drug for treating ADHD (see Hunt et al, Journal of the American Academy of Child Adolescent Psychiatry - 24, 1 January 1995). Although clonidine has been shown to be effective, it does, unfortunately, involve side-effects, the most serious of which is causing hypotension and a high level of sedation. While a measure of sedation can be useful in the treatment of hyperactive children, it does not assist in increasing attention span.

The present invention is based on the discovery that Lofexidine has usefulness in the treatment of ADHD without incurring the same level of side-effects as clonidine.

According to the present invention there is provided the use of Lofexidine or a non-toxic pharmaceutical acceptable salt thereof, in the preparation of a medicament for the treatment of ADHD.

Lofexidine, 2-[α-(2,6-dichlorophenoxy)ethyl)-Δ²-imidazole, has been used clinically for opiate detoxification (see Gold et al - Drug & Alcohol Dependency - 1981, 8, 307-315). Lofexidine is believed to work in opiate detoxification by reducing brain noradrenergic activity, thereby suppressing the withdrawal symptoms. The neurological reasons for ADHD are not, at present, known but it is possible that clonidine and lofexidine may alleviate the symptoms by acting on alpha 2 receptors in the brain, to affect the rate of release of noradrenaline and that activity may contribute to their usefulness in treating ADHD. However, because of the different activities noted in the case of clonidine and lofexidine, it is apparent that they act either in a different way on the same receptors, or perhaps on associated receptors.

Investigations with monkeys have shown that dose rates of the order of 0.005 mg to 0.02 mg per kilogram show an improvement in cognitive function with little sedation or blood pressure problems. This translates into a daily dose rate for adults of up to about 3 mg, e.g. about 0.5 to about 2.5 mg per day, typically about 1 to 2 mg per day. In the case of children, the daily dose rate is expected to be correspondingly smaller. The dose administered would, to a large extent, depend upon the method of administration.

The drugs can be administered in traditional form for oral administration, e.g. as tablets, lozenges, dragees and capsules. However, for the administration of the drug to children, which is likely to be its major use, it may be preferred to formulate the composition as a suppository, skin patch or oral liquid preparation such as a syrup. The drugs can also be administered parentally, e.g. by intramuscular or subcutaneous injection, using formulations in which the drug is employed in a saline or other pharmaceutically acceptable, injectable composition.

An advantage of lofexidine for these purposes is that it is water-soluble, especially at a slightly acid pH.

Preparations for suitable for paediatric use include aqueous syrups, which are preferably formulated with an acid constituent such as citric or ascorbic acid. Sweetners and flavourings are also desirable ingredients. In the case of liquid preparations, a typical concentration would be about 0.05 to 2 mg, e.g. 0.1 to 1 mg, per 5 ml of aqueous liquid. For example, if the daily dose is, say, about 1 mg per day, then an oral liquid formulation may contain 0.2 mg per 5 ml and five 5 ml spoonfuls would be administered throughout the day.

Another good method of administering the compositions of the invention is in the form of skin patches. A typical structure suitable for use as a skin patch is as follows.

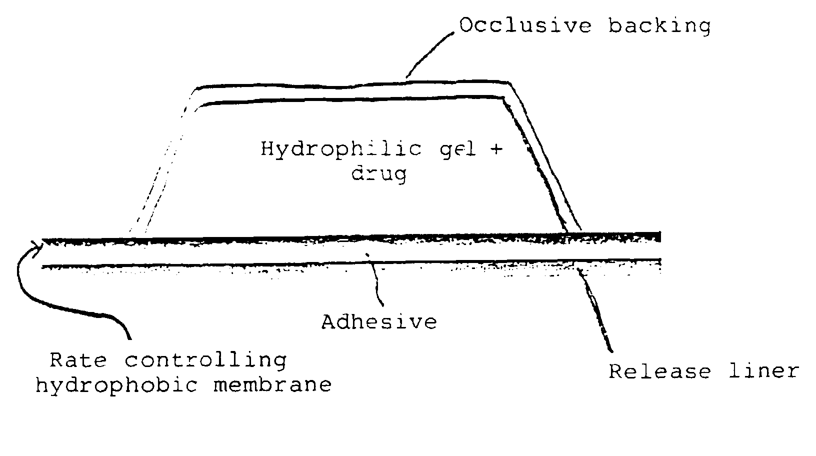

The concentration of the lofexidine in the hydrophilic gel layer is preferably calculated so as to deliver a dosage of about 0.05 to 2 mg per day.

Controlled release compositions may also be useful in the practice of tins invention. The controlled release composition may be designed to give an initial high dose of the active material and then a steady dose over an extended period of time, or a slow build up to the desired dose rate, or variations of these procedures.

Nasal spray compositions are also a useful way of administering the pharmaceutical preparations of this invention to patients such as children for whom compliance is difficult. Such formulations are generally aqueous and are packaged in a nasal spray applicator which delivers a fine spray of the composition to the nasal passages.

Suppositories are also a traditionally good way of administering drugs to children and can be used for the purposes of this invention. Typical bases for formulating suppositories include water-soluble diluents such as polyalkylene glycols and fats, e.g. cocoa oil and polyglycol ester or mixtures of such materials.

In the treatment of ADHD patients in accordance with the invention, lofexidine can be used alone or together with other active materials, For example, lofexidine can be used in conjunction with clonidine and/or with a related alpha 2 adrenergic agonist such as guanfacine. This can be beneficial because clonidine produces a quick but short acting benefit in improving the condition of a ADHD patients, while lofexidine with or without guanfacine or clonidine gives a prolongation of the effect. It is also believed that lofexidine may also be useful in conjunction with guanfacine on its own for extending the affect of the treatment over an extended period.

In the case of clonidine, this drug can be used to provide a quick effect, while lofexidine administered simultaneously or subsequently provides a longer-lasting control of ADHD. Dosage rates for clonidine in this embodiment are relatively low, e.g. 0.005 to 0.01 mg/kg, and may be administered by a skin patch to establish a low level of clonidine in the patient's blood. Lofexidine is also preferably administered at a low dosage level, preferably from about 0.008 to 0.015 mg/kg, e.g. about 0.01 mg/kg. Because lofexidine may, in some patients, cause a small degree of sedation, a stimulant may be incorporated in the medication or administered separately in conjunction with the lofexidine. Suitable stimulants include methyl phenidate, dexamphetamine or pemoline

Although the invention has been described primarily as a therapy for children, it can also be used for adults, although dosage rates may be different in the case of adults.

The following Examples show the effect of lofexidine alone administered to monkeys. In these tests, the monkeys used were aged specimens and were tested for attention span by repetitively carrying out a set task with and without treatment with lofexidine, administered parentally at the rate of 0.01 mg and 0.05 mg per kg per day. The tabulated results below show an improvement in attention span of up to about 60% with no apparent sedation and with little effect on blood pressure.

### Example 1

| Monkey Number | Task vehicle alone | Correct medicament | % improvement | Appearance after drug administration |
|---|---|---|---|---|
| 1 | 9 out of 30 | 26 out of 30 | 56.6 | calm, not pale |
| 2 | 23 out of 30 | 28 out of 30 | 17 | alert |
| | vehicle alone | medicament at 0.5 mg | | |
| 1 | 7 out of 15 | 26 out of 30 | 63.27 | sleepy |

Although the result for monkey 1 in the above test showed a slightly greater percentage improvement after administering five times the dosage in the first test, the subject was noticeably sleepy. This suggests that a dosage level of about 0.01 mg/kg is likely to be the optimum.

### Example 2

Two young monkeys who had become agitated because of a move to new quarters and developed 'ADHD like' behaviour were examined to determine the effect of lofexidine on this behaviour. Again, the monkeys were tested by giving them a repetitive task and noting the number of times when the task was carried out correctly. The monkeys were tested after injection of a saline solution of lofexidine at the rate of 0.01 mg/kg and after injection of the saline vehicle alone. The results are as follows:-

| Monkey Number | Task correct | | % improvement |
|---|---|---|---|
| | vehicle alone | medicament at 0.01 mg | |
| 3 | 14 out of 30 | 21 out of 30 | 23 |
| 4 | 11 out of 30 | 17 out of 30 | 20 |

In both cases, the monkeys appeared alert and non-sedated after administration of the medicament.

## Claims

1. Use of lofexidine or a non-toxic pharmaceutically acceptable salt thereof in the preparation of a medicament for the treatment of ADHD.

2. Use as claimed in claim 1 wherein the lofexidine is employed in an amount of from 0.005 to 0.02 mg/kg.

3. Use as claimed in claim 1 or 2 wherein the medicament additionally contains at least one further noradrenergic agonist selected from clonidine and guanfacine.

4. Use as claimed in any one of the preceding claims wherein the medicament is formulated as a controlled release preparation.

5. Use as claimed in any one of the preceding claims wherein the medicament is formulated as a suppository, skin patch, syrup or nasal spray.

6. Use as claimed in any one of the preceding claims wherein the medicament additionally contains a stimulant.

7. Use as claimed in claim 6 wherein the stimulant is methyl phenidate, dexamphetamine or pemoline.

## Patentansprüche

1. Verwendung von Lofexidin oder einem nicht-toxischen, pharmazeutisch akzeptablen Salz davon in der Herstellung eines Medikaments zur Behandlung von ADHD.

2. Verwendung nach Anspruch 1, worin das Lofexidin in einer Menge von 0,005 bis 0,02 mg/kg verwendet wird.

3. Verwendung nach Anspruch 1 oder 2, worin das Medikament zusätzlich mindestens einen weiteren noradrenergen Agonisten umfasst, ausgewählt aus Clonidin und Guanfacin.

4. Verwendung nach einem der vorhergehenden Ansprüche, worin das Medikament als ein Präparat, das kontrollierte Freisetzung ermöglicht, formuliert wird.

5. Verwendung nach einem der vorhergehenden Ansprüche, worin das Medikament als Zäpfchen, Hautpflaster, Sirup oder Nasenspray formuliert wird.

6. Verwendung nach einem der vorhergehenden Ansprüche, worin das Medikament zusätzlich ein Stimulans enthält.

7. Verwendung nach Anspruch 6, worin das Stimulans Methylphenidat, Dexamphetamin oder Pemolin ist.

## Revendications

1. Utilisation de lofexidine ou l'un de ses sels non-toxique à effet pharmaceutique pour préparer un médicament destiné à traiter l'ADHD.

2. Utilisation selon la revendication 1, dans laquelle, on emploie la lofexidine selon une quantité de 0,005 à 0,02 mg/kg.

3. Utilisation selon la revendication 1 ou 2, dans laquelle le médicament comprend en plus au moins un agoniste noradrénergique supplémentaire choisi parmi la clonidine et la guanfacine.

4. Utilisation selon l'une des revendications précédentes, dans laquelle on formule le médicament comme une préparation à effet retard.

5. Utilisation selon l'une des revendications précédentes, dans laquelle on formule le médicament sous forme de suppositoire, de timbre pour la peau, de sirop ou de vaporisateur nasal.

6. Utilisation selon l'une des revendications précédentes, dans laquelle le médicament comprend en plus un stimulant.

7. Utilisation selon la revendication 6, dans laquelle le stimulant est le méthylphénidate, la dexamphétamine ou la pémoline.
